(19) Europäisches Patentamt
European Patent Office
Office européen des brevets

(11) **EP 2 259 820 B1**

(12) **EUROPEAN PATENT SPECIFICATION**

(45) Date of publication and mention
of the grant of the patent:
**13.03.2013 Bulletin 2013/11**

(21) Application number: **09727336.1**

(22) Date of filing: **30.03.2009**

(51) Int Cl.:
*A61M 16/00* (2006.01)  *A61H 33/00* (2006.01)

(86) International application number:
**PCT/US2009/038820**

(87) International publication number:
**WO 2009/123981 (08.10.2009 Gazette 2009/41)**

(54) **LEAK-COMPENSATED PROPORTIONAL ASSIST VENTILATION**

PROPORTIONALE HILFSBEATMUNG MIT LECKKOMPENSATION

VENTILATION ASSISTÉE PROPORTIONNELLE À COMPENSATION DES FUITES

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **31.03.2008 US 41070 P**
**20.03.2009 US 408408**

(43) Date of publication of application:
**15.12.2010 Bulletin 2010/50**

(73) Proprietor: **Nellcor Puritan Bennett LLC**
**North Haven, Connecticut 06473 (US)**

(72) Inventors:
• **JAFARI, Mehdi, M.**
**Laguna Hills**
**CA 92653 (US)**

• **AVIANO, Jeffrey, K.**
**Escondido**
**CA 92026 (US)**
• **JIMENEZ, Rhomere**
**San Diego, CA 92154 (US)**
• **UPHAM, Gail, F.**
**Fallbrook**
**CA 92028 (US)**

(74) Representative: **Beyer, Andreas**
**Wuesthoff & Wuesthoff**
**Patent- und Rechtsanwälte**
**Schweigerstrasse 2**
**81541 München (DE)**

(56) References cited:
**WO-A-00/10634      WO-A-00/45880**
**WO-A-2004/084980   DE-A1- 19 808 543**
**US-A- 5 148 802**

EP 2 259 820 B1

**Description**

International Patent Application Publication WO 00/10634 discloses non-invasively determining the elastance of a patient's respiratory system by using a flow estimation which self-corrects for leak estimation errors. International Patent Application Publication WO 2004/084980 discloses detecting leaks in ventilation systems by taking the difference of measured sequences of values of pressure and flow in two successive respiratory cycles. German Patent Application DE 198 08 543 discloses determining mechanical properties of a respiratory system of a patient connected to a respirator, wherein a short-term occlusion of the patient's airways is performed during an inspiration phase and/or an expiration phase.

**INTRODUCTION**

In mechanical ventilation, proportional assist (PA) refers to a type of ventilation in which the ventilator acts as an inspiratory amplifier that provides pressure support based on the patient's effort. The degree of amplification (the "support setting") is set by an operator, for example as a percentage based on the patient's effort. In one implementation of PA ventilation, the ventilator may continuously monitor the patient's instantaneous inspiratory flow and instantaneous net lung volume, which are indicators of the patient's inspiratory effort. These signals, together with ongoing estimates of the patient's lung compliance and lung resistance, allow the ventilator to compute the instantaneous pressure at a point in the ventilation circuit that assists the patient's inspiratory muscles to the degree selected by the operator as the support setting.

PA ventilation relies on certain physiological principles. The act of inspiration requires the patient's inspiratory muscles to develop a pressure gradient between the mouth and the alveoli sufficient to draw in breathing gas and inflate the lungs. Some of this pressure gradient is dissipated as gas travels through the artificial airway and the patient's conducting airways, and some of the pressure gradient is dissipated in the inflation of the lungs and thorax. Each element of pressure dissipation is characterized by a measurable property: the resistance of the artificial and patient airways, and the compliance (or elastance) of the lung and thorax.

The ventilator providing PA ventilation uses specific information, including resistance of the artificial airway, resistance of the patient's airways, lung compliance, instantaneous inspiratory flow and net lung volume, and the support setting to compute the instantaneous pressure to be applied at the wye. The ventilator may estimate patient lung resistance and lung compliance, for example approximately every four to ten breaths. In one implementation, every computational cycle (e.g., 5 milliseconds), the ventilator estimates lung flow, based on an estimate of circuit flow, and lung volume, based on the integral value of estimated circuit flow.

PA ventilation begins inspiratory assist when flow (generated by the patient's inspiratory muscles) is detected. If the patient ceases inspiration, the assist also ceases. Once inspiratory flow begins, the ventilator monitors instantaneous flow and volume and applies the pressure calculated to deliver a proportion (determined by the support setting) of the total demand as determined by the patient's inspiratory effort. In Tube Compensation (TC) ventilation, the ventilator monitors instantaneous flow and volume and applies the pressure calculated to overcome a proportion (determined by the support setting) of the pressure losses dissipated across the resistance of the artificial airways (e.g., endotracheal tube).

The lung compliance and lung resistance of a patient may be collectively referred to as the respiratory mechanics of the lung or, simply, the patient's respiratory mechanics. Because PA relies on the patient's respiratory mechanics, more accurate determination of respiratory mechanics is essential to performance of the ventilator when providing PA.

**Leak-Compensated Proportional Assist Ventilation**

This disclosure describes systems and methods for compensating for leakage when during delivery of gas to a patient from a medical ventilator in a proportional assist (PA) mode. The technology described herein includes systems and methods that compensate the delivery of PA ventilation for leakage in the patient circuit by using leak-compensated lung flows as well as respiratory mechanics (lung compliance and lung resistance) estimated in a manner that compensates for elastic and inelastic leaks from the ventilation system.

In part, this disclosure describes a method of compensating for leakage in a ventilation system during delivery of gas from a medical ventilator providing PA to a patient. The method includes monitoring an instantaneous flow in the ventilation system based on one or more measurements of pressure and flow in ventilation system. Leakage from the system is modeled as a first leakage component through a first orifice of a fixed size and a second leakage component through a second orifice of a varying size, in which the first and second leakage components are different functions of instantaneous pressure in the ventilation system. A leak-compensated instantaneous lung flow of gas inhaled or exhaled by the patient is estimated based on the one or more measurements, the first leakage component and second leakage component. The leak-compensated lung flow and a predetermined respiratory mechanics model are used to estimate a leak-compensated lung compliance and a leak-compensated lung resistance. A pressure to be delivered to the patient is then calculated based on the leak-compensated lung flow, the leak-compensated lung compliance and the leak-compensated lung resistance.

This disclosure describes a method of compensating for leakage in a ventilation tubing system during delivery of gas from a medical ventilator to a patient. The method includes receiving a support setting identifying an amount of proportional assistance to provide to the patient. An inelastic leakage in the ventilation system is identified as a first function of at least one of a pressure measurement and a flow measurement in the ventilation system. An elastic leakage in the ventilation system is also identified as a second function of at least one of the pressure measurement and the flow measurement in the ventilation system. The circuit compliance and circuit resistance of the ventilation tubing system is then used along with estimated lung compliance of the patient and estimated lung resistance of the patient based on the inelastic leakage, the elastic leakage, the circuit compliance, circuit resistance and the at least one of the pressure measurement and the flow measurement in the ventilation system. Ventilation is then delivered to the patient based on estimated patient effort and the support setting, in which the patient effort is determined from estimated lung flow using the inelastic leakage, the elastic leakage, the lung compliance and the lung resistance.

The disclosure also describes a pressure support system that includes a pressure generating system adapted to generate a flow of breathing gas and a ventilation tubing system including a patient interface device for connecting the pressure generating system to a patient. One or more sensors are operatively coupled to the pressure generating system or the ventilation system, in which each sensor capable of generating an output indicative of a pressure of the breathing gas. A leak estimation module is provided that identifies leakage in the ventilation system and compensates the calculation of lung flow for the estimated leakage in the system. A respiratory mechanics calculation module is further provided that generates a leak-compensated lung compliance and a leak-compensated lung resistance based on the leakage and at least one output indicative of a pressure of the breathing gas. The system further includes a proportional assistance ventilation module that causes the pressure generating system to provide ventilation to the patient based on patient effort and a support setting, in which the patient effort is determined from estimated lung flow using the leakage, the leak-compensated lung compliance and the leak-compensated lung resistance.

The disclosure further describes a PA ventilation controller for a medical ventilator. The controller includes a microprocessor, a module that compensates calculations of lung compliance and lung resistance based on instantaneous elastic leakage and instantaneous inelastic leakage of breathing gas from a ventilation system, and a pressure control module that provides proportional assist ventilation based on the compensated lung compliance and lung resistance.

These and various other features as well as advantages which characterize the systems and methods described herein will be apparent from a reading of the following detailed description and a review of the associated drawings. Additional features are set forth in the description which follows, and in part will be apparent from the description, or may be learned by practice of the technology. The benefits and features of the technology will be realized and attained by the structure particularly pointed out in the written description and claims hereof as well as the appended drawings.

It is to be understood that both the foregoing general description and the following detailed description are exemplary and explanatory and are intended to provide further explanation of the invention as claimed. The invention is defined by the independent claims. The disclosed methods are not claimed

## BRIEF DESCRIPTION OF THE DRAWINGS

The following drawing figures, which form a part of this application, are illustrative of described technology and are not meant to limit the scope of the invention as claimed in any manner, which scope shall be based on the claims appended hereto.

FIG. 1 illustrates an embodiment of a ventilator connected to a human patient.

FIG. 2 schematically depicts example systems and methods of ventilator control.

FIG. 3 illustrates an embodiment of a method of compensating for leakage in a ventilator providing pressure assistance to a patient.

FIG. 4 illustrates an embodiment of a method for providing a leak-compensated PA breath to a patient.

FIG. 5 illustrates an embodiment of a method for estimating respiratory mechanics of patient that utilizes a respiratory mechanics maneuver.

FIG. 6 illustrates an embodiment of a method for dynamically estimating respiratory mechanics of patient.

FIG. 7 illustrates a functional block diagram of modules and other components that may be used in an embodiment of ventilator that compensates for elastic and rigid orifice sources of leaks when performing PA ventilation.

## DETAILED DESCRIPTION

Although the techniques introduced above and discussed in detail below may be implemented for a variety of medical devices, the present disclosure will discuss the implementation of these techniques in the context of a medical ventilator providing pressure assist (PA) ventilation to a human patient. The reader will understand that the technology described in the context of a medical ventilator for human patients could be adapted for use with other systems such as ventilators for non-human patients and general gas transport systems in which leaks may cause a degradation of performance.

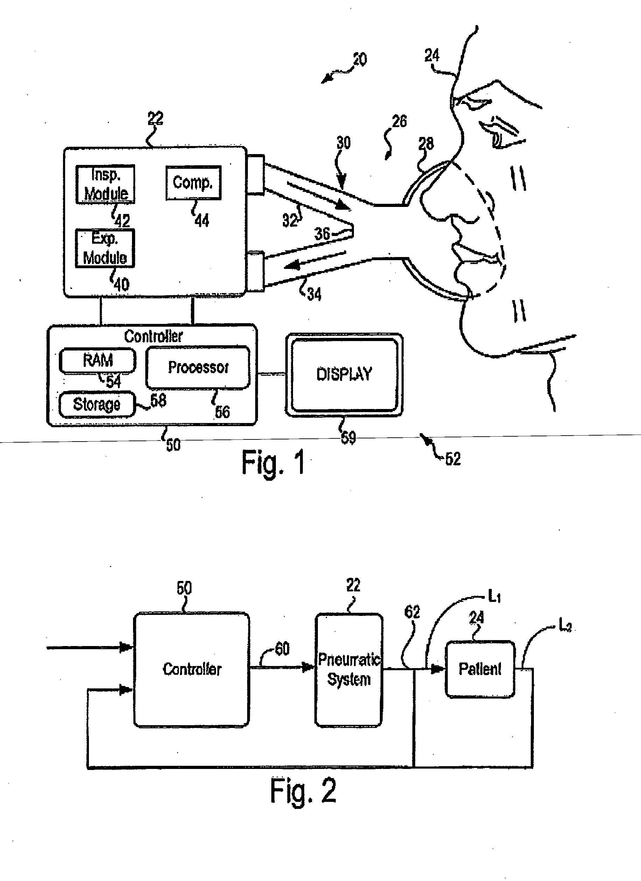

FIG. 1 illustrates an embodiment of a ventilator **20** connected to a human patient **24**. Ventilator **20** includes a pneumatic system **22** (also referred to as a pressure generating system **22**) for circulating breathing gases to and from patient **24** via the ventilation tubing system **26**, which couples the patient to the pneumatic system via physical patient interface **28** and ventilator circuit **30**. Ventilator circuit **30** could be a dual-limb or single-limb circuit for carrying gas to and from the patient. In a dual-limb embodiment as shown, a wye fitting **36** may be provided as shown to couple the patient interface **28** to the inspiratory limb **32** and the expiratory limb **34** of the circuit **30**.

The present systems and methods have proved particularly advantageous in noninvasive settings, such as with facial breathing masks, as those settings typically are more susceptible to leaks. However, leaks do occur in a variety of settings, and the present description contemplates that the patient interface may be invasive or non-invasive, and of any configuration suitable for communicating a flow of breathing gas from the patient circuit to an airway of the patient. Examples of suitable patient interface devices include a nasal mask, nasal/oral mask (which is shown in FIG. 1), nasal prong, full-face mask, tracheal tube, endotracheal tube, nasal pillow, etc.

Pneumatic system **22** may be configured in a variety of ways. In the present example, system **22** includes an expiratory module **40** coupled with an expiratory limb **34** and an inspiratory module **42** coupled with an inspiratory limb **32**. Compressor **44** or another source(s) of pressurized gas (e.g., air and oxygen) is coupled with inspiratory module **42** to provide a gas source for ventilatory support via inspiratory limb **32**.

The pneumatic system may include a variety of other components, including sources for pressurized air and/or oxygen, mixing modules, valves, sensors, tubing, accumulators, filters, etc. Controller **50** is operatively coupled with pneumatic system **22**, signal measurement and acquisition systems, and an operator interface **52** may be provided to enable an operator to interact with the ventilator (e.g., change ventilator settings, select operational modes, view monitored parameters, etc.). Controller **50** may include memory **54**, one or more processors **56**, storage **58**, and/or other components of the type commonly found in command and control computing devices.

The memory **54** is computer-readable storage media that stores software that is executed by the processor **56** and which controls the operation of the ventilator **20**. In an embodiment, the memory **54** comprises one or more solid-state storage devices such as flash memory chips. In an alternative embodiment, the memory **54** may be mass storage connected to the processor **56** through a mass storage controller (not shown) and a communications bus (not shown). Although the description of computer-readable media contained herein refers to a solid-state storage, it should be appreciated by those skilled in the art that computer-readable storage media can be any available media that can be accessed by the processor **56**. Computer-readable storage media includes volatile and nonvolatile, removable and non-removable media implemented in any method or technology for storage of information such as computer-readable instructions, data structures, program modules or other data. Computer-readable storage media includes, but is not limited to, RAM, ROM, EPROM, EEPROM, flash memory or other solid state memory technology, CD-ROM, DVD, or other optical storage, magnetic cassettes, magnetic tape, magnetic disk storage or other magnetic storage devices, or any other medium which can be used to store the desired information and which can be accessed by the computer.

As described in more detail below, controller **50** issues commands to pneumatic system **22** in order to control the breathing assistance provided to the patient by the ventilator. The specific commands may be based on inputs received from an operator, the patient **24**, the pneumatic system **22** and sensors, the operator interface **52** and/or other components of the ventilator. In the depicted example, operator interface includes a display **59** that is touch-sensitive, enabling the display to serve both as an input and output device.

FIG. 2 schematically depicts exemplary systems and methods of ventilator control. As shown, controller **50** issues control commands **60** to drive pneumatic system **22** and thereby circulate breathing gas to and from patient **24**. The depicted schematic interaction between pneumatic system **22** and patient **24** may be viewed in terms of pressure and/or flow "signals." For example, signal **62** may be an increased pressure which is applied to the patient via inspiratory limb **32**. Control commands **60** are based upon inputs received at controller **50** which may include, among other things, inputs from operator interface **52**, and feedback from pneumatic system **22** (e.g., from pressure/flow sensors) and/or sensed from patient **24**.

In an embodiment, before the respiratory mechanics of a patient can be determined, the mechanics of the ventilation tubing system may be determined. For example, when modeling the delivery of gas to and from a patient **24** via a closed-circuit ventilator, one simple assumption is that compliance of the ventilator circuit **30** (the "circuit compliance") is fixed and that all gas injected into the ventilator circuit **30** that does not exit the circuit **30** via the expiratory limb **34** (in a dual-limb embodiment) fills the circuit as well as the patient's lungs and causes an increase in pressure. As gas is injected ($L_1$), the lung responds to the increased gas pressure in the circuit **30** by expanding. The amount the lung expands is proportional to the lung compliance and is defined as a function of gas pressure differential (e.g., lung compliance = volume delivered/pressure difference). As discussed in greater detail below, this assumption is not valid when leaks are present.

The term circuit compliance is used to refer to the relationship between the amount the pressure in the ventilator circuit **30** (or ventilator circuit **30** and attached patient interface **28**, depending on how the compliance is determined) and changes in volume delivered into the circuit. In an embodiment, the circuit compliance may be estimated by pressurizing the ventilator circuit **30** (or circuit **30** and interface **28** combination) when flow to the patient is blocked and measuring the volume of additional gas introduced to cause the pressure change (compliance = volume delivered/pressure difference).

The term circuit resistance is used to refer to the amount the pressure changes between two sites upstream and downstream the ventilator circuit as a function of volumetric flow rate through that circuit. Circuit resistance may be modeled as a two-parameter function of flow and several methods for modeling and calculating circuit resistance are known in the art. For example, in an embodiment, the circuit resistance may be estimated by passing several fixed flow rates through the circuit and measuring the pressure difference between certain upstream and downstream sites and finding the best curve fit to the collected data.

Methods of determining circuit compliance and circuit resistance (such as those described above) may be executed by the operator prior to attaching the patient to the ventilator as part of the set up of the ventilator **20** to provide therapy. Other methods of determining circuit compliance and/or resistance are also known and could be adapted for use with the disclosed leak-compensation systems and methods described herein.

In many cases, it may be desirable to establish a baseline pressure and/or flow trajectory for a given respiratory therapy session. The volume of breathing gas delivered to the patient's lung ($L_1$) and the volume of the gas exhaled by the patient ($L_2$) are measured or determined, and the measured or predicted/estimated leaks are accounted for to ensure accurate delivery and data reporting and monitoring. Accordingly, the more accurate the leak estimation, the better the baseline calculation of delivered and exhaled flow rates and volumes.

Errors may be introduced due to leaks in the ventilation tubing system **26.** The term ventilation tubing system **26** is used herein to describe the ventilator circuit **30**, any equipment attached to or used in the ventilator circuit **30** such as water traps, monitors, drug delivery devices, etc. (not shown), and the patient interface **28**. Depending on the embodiment, this may include some equipment contained in the inspiration module **42** and/or the expiration module **40**. When referring to leaks in or from the ventilation tubing system **26**, such leaks include leaks within the tubing system **26** and leaks where the tubing system **26** connects to the pressure generator **22** or the patient **24**. Thus, leaks from the ventilation tubing system **26** include leaks from the ventilator circuit **30**, leaks from the patient interface **28** (e.g., masks are often provided with holes or other pressure relief devices through which some leakage may occur), leaks from the point of connection of the patient interface **28** to the patient **24** (e.g., leaks around the edges of a mask due to a poor fit or patient movement), and leaks from the point of connection of the patient interface **28** to the circuit **30** (e.g., due to a poor connection between the patient interface **28** and the circuit **30**).

For the purpose of estimating how a leak flow rate changes based on changes in pressure in the ventilation tubing system **26**, the instantaneous leak may be modeled as a leak through a single rigid orifice or opening of a fixed size in which that size is determined based on comparing the total flow into the inspiratory limb **32** and out of the expiratory limb **34**. However, this leak model does not take into account any elastic component of leak source(s) in the system **26**, that is how much of the area of any of the holes or openings in the ventilation tubing system **26** through which leakage occurs may change due to an increase or decrease in pressure.

It has been determined that not accounting for elastic leakage from the ventilation tubing system **26** can cause many problems. First, if only the inelastic/fixed orifice model is used to estimate leak, the subsequent errors caused by ignoring the elastic effects of any actual leaks end up generating inaccurate estimates of flow rates into the lung. This can cause the ventilator **20** to estimate gas volume delivered into the lung inaccurately when, in fact, the elastic leaks in the system **26** have let more gas escape than estimated. Second, if the elasticity of the leak source is ignored, any other calculation, estimate, or action that the ventilator **20** may perform which is affected by the leak estimate will be less accurate.

In the systems and methods described herein, the provision of PA ventilation is made more accurate by compensating for tubing system leakage. In the embodiments described herein fixed (rigid) and elastic components of the system leakage are used when determining the lung flow, net lung volume, lung compliance and lung resistance of the patient. This results in a more accurate determination of lung compliance and lung resistance and, therefore, ventilation of patients based on respiratory mechanics. While the systems and methods are presented in the context of specific leakage models, the technology described herein could be used to compensate the respiratory mechanics determined by any model for leakage using any type of mechanical ventilator or other device that provides gas.

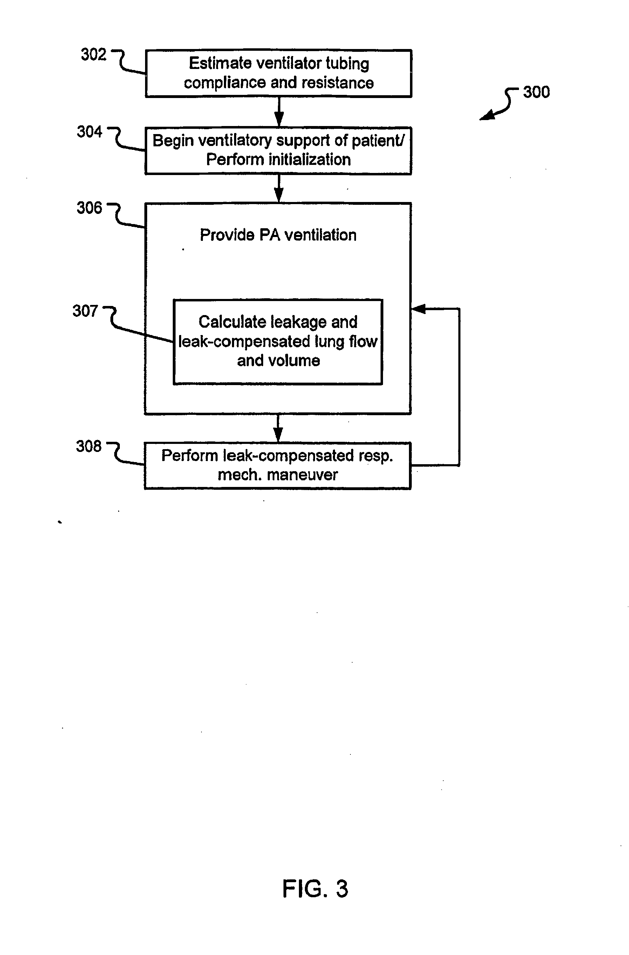

FIG. 3 illustrates an embodiment of a method of compensating PA ventilation for leakage during delivery of gas from a medical ventilator to a patient. In the method **300** shown, a medical ventilator such as that described above with reference to FIGS. 1 and 2 is used to provide PA ventilation to a patient.

The method **300** illustrated starts with a circuit compliance and resistance operation **302**. In that operation **302**, the ventilator circuit compliance and resistance are estimated. In an embodiment, this may be performed prior to connecting the ventilator to the patient (as previously described). Alternatively, it may be dynamically determined periodically throughout the delivery of ventilatory support to the patient.

After the circuit compliance and resistance have been determined, the ventilator is connected to the patient and an initialization operation **304** is performed. In the initialization operation **304** the ventilator operates for an initialization or startup period in order to generate an initial estimate of lung compliance and lung resistance. If the ventilator already has some knowledge of the respiratory mechanics of the patient (e.g., the respiratory mechanics have been recently determined during provision of a different type of ventilation or an operator has provided initial settings for lung compliance and resistance), this operation **304** may be automatically or manually omitted in favor of the previously determined values.

A description of an embodiment of the initialization operation **304** is as follows. Because the ventilator does not know the patient's mechanics when the PA breath type is selected, it performs a startup routine to obtain initial data. In an embodiment, upon startup the ventilator delivers some number (e.g., two, four, etc.) of consecutive PA breaths, each of which includes an end-inspiratory maneuver that yields estimates of the patient's resistance and compliance. Using four training breaths for the initialization operation **304** as an example, the first breath is delivered using a predicted resistance for the artificial airway and conservative estimates for patient resistance and compliance. The predicted values may be determined based on known characteristics of the patient, such as based on the patient's ideal body weight (IBW), height, gender, age, physical condition, etc. Each of the following three PA breath's averages stepwise decreased physiologic values with the estimated resistance and compliance values from the previous breath, weighting earlier estimates less with each successive breath, and yielding more reliable estimates for lung resistance and lung compliance.

In an embodiment of the method **300**, a leakage estimate may also be done prior to the initialization operation **304**. Prior determination of leak parameters allows for estimates of respiratory mechanics to be made. This may include delivering pressure-regulated breaths with specific settings or performing specific "leak maneuvers", that is a specified set of controlled actions on the part of the ventilator that allow leakage parameters to be identified and quantified, such as interrupting the therapeutic delivery of respiratory gas and holding or changing the pressure and flow, so that data concerning the leakage of the system during the controlled actions may be obtained. For example, a leak maneuver may include periodically holding the pressure and flow in the circuit constant while determining (based on a comparison of the measured flow into the inspiratory limb and the measured flow out of the expiratory limb via the exhalation valve) the net leakage from the system. In an embodiment, such a leak maneuver may be performed during specific parts of the respiratory phase, e.g., at the end of the expiratory phase. In yet another embodiment, a sequence of pressure-based test breaths is delivered with specific settings to determine leak parameters prior to execution of test breaths for respiratory mechanics determinations.

After the initialization operation **304**, the ventilator provides ongoing PA ventilation to the patient in a PA ventilation operation **306**. PA ventilation begins inspiratory assist when an inspiratory trigger is detected such as leak-compensated lung flow (generated by the patient's inspiratory muscles) exceeds a set sensitivity threshold. If the patient ceases inspiration, the assist also ceases. Once inspiratory flow begins, the ventilator monitors instantaneous flow and volume and applies the pressure calculated to deliver a proportion (determined by the support setting) of the total demand as determined by the patient's inspiratory effort.

During PA ventilation, pressure assistance is provided to the patient based on the patient's effort and the operator-selected support setting. PA ventilation determines how much to assist the patient's efforts based on one or more equations of motion used to describe the mechanics of the lung. Such equations use respiratory mechanics of the patient, e.g., lung resistance and lung compliance, and the circuit when calculating how much flow and/or pressure to provide. One example of an equation of motion for use in determining the pressure to provide during PA ventilation is as follows:

$$P_{aw}(t) = E \int Q \, dt + Q \, R - P_m(t)$$

where $P_{aw}$ is the pressure measured at the patient interface, $P_m$ is the pressure generated by the inspiratory muscles of the patient which is may be used as the index of the patient's effort, E is the lung elastance (which is the inverse of lung compliance, i.e., E = 1/C), Q is the instantaneous leak-compensated lung flow and R is the lung resistance. By manipulating this equation slightly, an equation for the amount of desired pressure assistance at the patient interface to provide can be obtained:

$$P_{aw}(t) = k \, E \int Q \, dt + k \, Q \, R$$

where k is the % support setting based on the patient effort, $P_m$. The equations above are one example of how PA ventilation may be provided based on patient effort. PA ventilation is known in the art and any suitable technique or set of equations for determining the assistance to provide may be used.

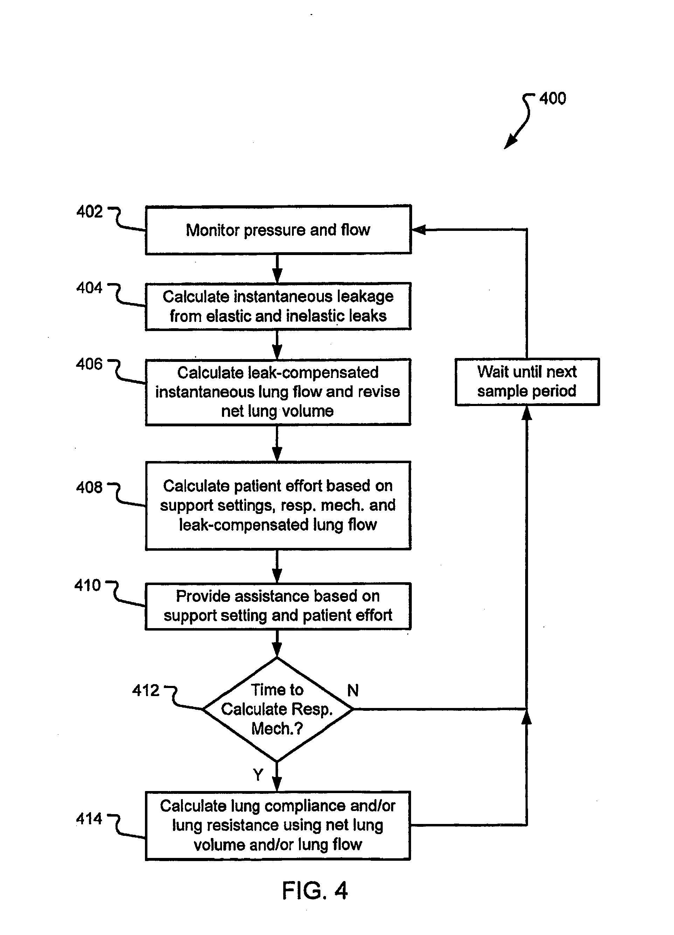

As described above, the patient's effort is determined based on the pressure and leak-compensated lung flow in the circuit. In order to compensate for leakage in the circuit, in the method **300** shown the PA ventilation operation **306** includes the ongoing calculation of leakage while providing ventilation, as illustrated by the leakage calculation/ compensation operation **307**. As discussed in greater detail below with reference to FIG. 4, the leakage is calculated and the leak-compensated values for lung flow, current lung volume and net delivered lung flow may be determined taking into account the calculated leakage.

The method **300** also includes periodically or randomly performing a respiratory mechanics maneuver in order to recalculate the lung compliance and lung resistance of the patient. In an embodiment, the respiratory mechanics maneuver operation **308** is performed randomly once every four to ten PA breaths.

The respiratory mechanics maneuver operation **308** compensates for leakage as determined by the leakage calculation/compensation operation **307** when calculating the respiratory mechanics for the patient. This may include stabilizing the pressure and flow in the patient circuit based on the calculated leakage at that pressure so that the flow into the patient circuit is approximately equal to the calculated amount of gas leaking from the patient circuit at that pressure; a period of stable pressure during which there is no flow into or out of the patient's lungs even though there is leakage flow in the ventilator tubing system. The respiratory mechanics maneuver operation **308** may further include using leak-compensated values of lung flow and lung volume when determining the respiratory mechanics.

The newly determined values of lung compliance and lung resistance may be averaged, low-pass filtered or otherwise combined with the previously determined values. These revised values are then stored for use in later delivery of PA ventilation and the ventilator returns to providing PA ventilation to the patient.

FIG. 4 illustrates an embodiment of a method for providing a leak-compensated PA breath to a patient. In an embodiment, the method **400** corresponds to the operations performed during the PA ventilation operation **306** and the respiratory mechanics maneuver operation **308** discussed with reference to FIG. 3. In the embodiment of the method **400** illustrated, the operations occur repeatedly while the ventilator is providing PA ventilation, such as once a sample period or computation cycle.

During PA ventilation, the pressure and flow and other parameters of the system are monitored, illustrated by the monitoring operation **402**. In an embodiment, the monitoring operation **402** collects data including the instantaneous pressure and/or flow at or indicative of one or more locations in the ventilation tubing system. Depending upon how a particular leak model is defined, the operation **402** may also include making one or more calculations using data from pressure and flow measurements taken by the sensors. For example, a model may require a flow measurement as observed at the patient interface even though the ventilation system may not have a flow sensor at that location in the ventilation tubing system. Thus, a measurement from a sensor or sensors located elsewhere in the system (or data from a different type of sensor at the location) may be mathematically manipulated in order to obtain an estimate of the flow observed at the patient interface in order to calculate the leak using the model.

The data obtained in the monitoring operation **402** is then used to calculate leakage from the ventilator tubing system in a leakage calculation operation **404**. In an embodiment, the leakage calculation operation **404** uses the data obtained in the monitoring operation **402**, e.g., some or all of the instantaneous pressure and flow data collected during the monitoring operation **402** as well as information about the current respiratory phase (inhalation or exhalation).

The leakage calculation operation **404** calculates an instantaneous leakage flow or volume for the sample period. The instantaneous leakage is calculated using a mathematical formula that has been previously determined. Any leakage model, now known or later developed, may be used. In an embodiment, the mathematical formula is a leakage model that separates the leak into the sum of two leak components, inelastic leak and elastic leak, in which each component represents a different relationship between the quantity of leakage from the ventilation system and the measured current/instantaneous pressure and/or flow of gas in the ventilation system. As discussed above, the inelastic leak may be modeled as the flow through a rigid orifice of a fixed size while the elastic leak may be modeled as the flow through a different orifice of a size that changes based on the pressure (or flow) of the gas in the ventilation system.

An example of a method and system for modeling leak in a ventilation system as a combination of an elastic leak component and an inelastic leak component can be found in commonly-assigned U.S. Provisional Patent Application Serial Number 61/041,070, filed March 31, 2008, titled VENTILATOR LEAK COMPENSATION. The VENTILATOR LEAK COMPENSATION represents one way of characterizing the leak from a ventilation system as a combination of elastic and inelastic components. Other methods and models are also possible and may be adapted for use with this technology.

The mathematical formula used to calculate leakage may contain several parameters that are empirically determined and that may be periodically or occasionally revised in order to maintain the accuracy of the leakage estimate. For example, in an embodiment the parameters of a leakage formula include a first constant associated with the rigid orifice and a second constant associated with the variable-sized orifice. At various times during ventilation, the calculated leakage may be checked against a measured leakage and, if the estimate is significantly different from the measured leakage, the constants may be revised. This revision of the parameters in a leakage formula may be done as part of the leakage calculation operation **404** or may be done as a separate operation (not shown) that may, or may not, be performed every sample period.

The term instantaneous is used herein to describe a determination made for any particular instant or sampling period based on the measured data for that instant. For example, if a pressure measurement is taken every 5 milliseconds (sample period), the pressure measurement and the leakage model can be used to determine an instantaneous leak flow based on the instantaneous pressure measurement. With knowledge of the length of the sample period, the instantaneous flow may then be used to determine an instantaneous volume of gas leaking out of the circuit during that sample period. For longer periods covering multiple sample periods the instantaneous values for each sample period may be summed to obtain a total leakage volume. If a measurement is also the most recent measurement taken, then the instantaneous value may also be referred to as the current value.

After the current leak has been calculated, the method **400** further estimates the leak-compensated instantaneous lung flow to or from the patient in a lung flow estimation operation **406**. The estimated lung flow is compensated for the leak flow calculated in the instantaneous leak calculation operation **404** so that it represents a more accurate estimate of the actual flow into (or out of depending on the point of view and period selected) the lungs of the patient.

In the embodiment illustrated, the leak-compensated net lung volume is also calculated as part of the lung flow estimation operation **406**. In an embodiment, this may be performed by maintaining a running summation of net flow into/out of the lung over the period of a breath. For example, upon triggering inhalation, the ventilator may set a variable corresponding to net lung volume to zero and, each sample period, update this net lung volume to include the detected leak-compensated instantaneous lung flow delivered to the patient during that sample period.

In the PA ventilation method **400** illustrated, the leak-compensated lung flow or net volume is then used along with the support setting and the previously determined lung resistance and lung compliance (which were themselves determined using leak-compensated lung flows and net lung volumes) to calculate the amount of assistance to provide during ventilation.

The PA ventilation method **400** also includes calculating the patient effort in effort calculation operation **408**. As described above, the operation **408** may use one or more equations that relate the amount of pressure to calculate the patient's effort based on the instantaneous lung flow, current lung volume, lung compliance, lung resistance, support setting and other factors such as circuit compliance and resistance. Various methods are known in the art for calculating patient effort that use one or more respiratory mechanics and other parameters measurable while providing ventilatory support. Any such method for determining patient effort, now known or later developed, may be used herein.

The appropriate amount of ventilation is then provided in a ventilation operation 410. In this operation, the patient effort calculated above and the support setting are used to determine how much assistance to provide. Of course, if there is no patient effort (i.e., during the expiratory phase), no ventilation is provided. In this case, the appropriate ventilation may be providing a predetermined positive end expiratory pressure (PEEP) level. Depending on the equations used, the effort calculation operation **408** may not be technically necessary as the amount of ventilation to provide may be determined directly from the monitored data and the previously determined values of lung compliance and resistance using an appropriate algorithm.

The PA ventilation method **400** also periodically determines the respiratory mechanics from the leak-compensated lung parameters, which is illustrated by the determination operation **412**. For example, in an embodiment respiratory mechanics may be calculated on a fixed or random schedule or calculated in response to an explicit operator command. In addition, depending on the respiratory mechanics determination method used, there may be a requirement that the respiratory mechanics calculation (using data collected during the breath) be performed at a certain point within the patient's respiration such as at the end of the inspiratory phase or the end of the expiratory phase or after the completion of a specific maneuver (e.g., an Inspiratory Hold Maneuver). The respiratory mechanics calculation includes the performance a respiratory mechanics "maneuver," that is a specified set of controlled actions on the part of the ventilator. In an embodiment, the maneuver includes interrupting the therapeutic delivery of respiratory gas for a period of time and monitoring and/or changing the pressure and flow, so that data concerning the response of the patient's lung to the controlled actions may be obtained. An example of a respiratory mechanics maneuver is provided below with reference to the static determination of lung compliance.

If it is time to calculate respiratory mechanics, a calculate respiratory mechanics operation **414** is performed. The calculate respiratory mechanics operation **414** may also include performing the appropriate maneuver, as necessary to obtain the data for the respiratory mechanics model being used. In the operation **414**, the necessary data for the respiratory mechanics model being used is obtained and the respiratory mechanics parameters are estimated.

In the calculate respiratory mechanics operation **414** leak-compensated values are used to estimate the respiratory mechanics. For example, if the respiratory mechanics model being used requires a total delivered lung volume, the leak-compensated lung volume is used. Likewise, if an instantaneous lung flow is required, a leak-compensated instantaneous lung flow is used in generating the estimate.

The method **400** is then repeated every computational cycle or sample period, as illustrated by the feedback loop, so that the instantaneous lung flow and net lung flow are continuously determined and, when appropriate, the respiratory mechanics are recalculated based on the current leak-compensated data. In an alternative embodiment, the leak-compensation of lung flow and net delivered lung volume may be performed as part of the calculate respiratory mechanics operation **414** in order to reduce the number of calculations a processor must perform every cycle.

The following is a discussion of two embodiments of methods for compensating the estimation of respiratory mechanics in the presence of leaks. The first embodiment is that of applying leak compensation to a static compliance and resistance determination. The second embodiment is that of applying leak compensation to a dynamic compliance determination.

## Leak-Compensation of Static Determination of Lung Compliance

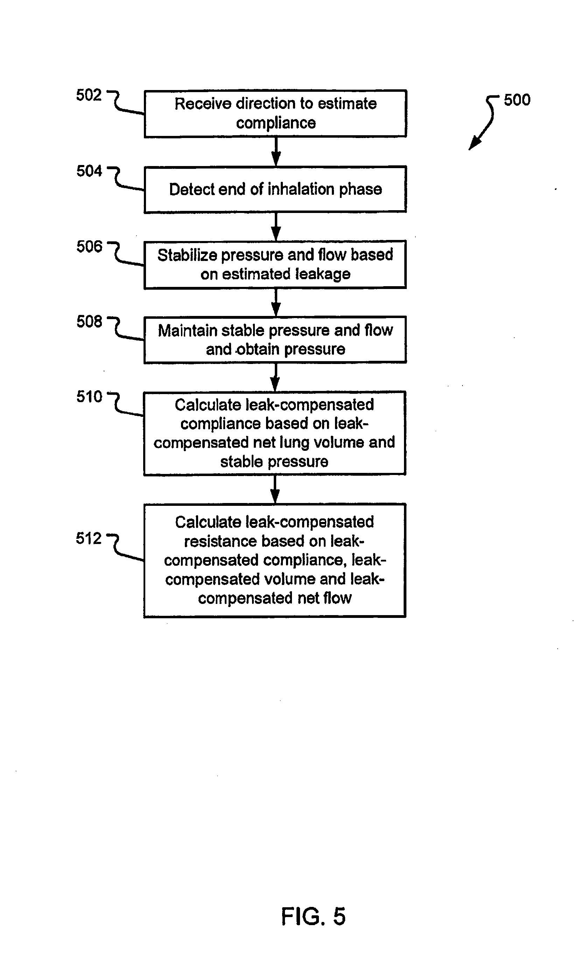

FIG. 5 illustrates an embodiment of a method for estimating respiratory mechanics of patient that utilizes a respiratory mechanics maneuver. In the embodiment shown, the ventilator is providing respiratory gas to a patient in accordance with some mode of operation such as a mandatory mode or a pressure assist mode (e.g., a mandatory volume-controlled (VCV) inspiration under square waveform setting or a mandatory pressure-controlled (PCV) inspiration with specific settings, or execute an inspiratory hold at the end of a PA inspiration), as is well known in the art. During operation, an operator command to estimate the compliance of the patient is received in a receive command operation **502**. In an embodiment, the command may be entered by the operator of the ventilator via selection of a button or other interface element on a user interface of the ventilator. In an alternative embodiment, the ventilator may perform the method **500** automatically such as periodically, randomly or upon the detection of predetermined respiratory event.

In the embodiment shown, the system performs a respiratory maneuver which includes the forced imposition of a stable period at the end of an inspiratory phase so that there is no flow delivery to or from the patient's lung. The method **500** includes a delay until the next end of an inspiratory phase is detected, as illustrated by detect end of inspiratory phase operation **504**.

When the end of the inspiratory phase is detected, a stabilization operation **506** is performed. In an embodiment, the operation **506** includes stabilizing the pressure and flow in the patient circuit so that there is no flow into or out of the patient's lungs at the point in which the lungs have taken a breath and thus are expanded with a known volume of gas (as determined during normal operation of the ventilator as discussed above with reference to the leak-compensated lung volume).

The stabilization operation **506** and the maintain stable condition operation **508** (discussed below) may sometimes be referred to collectively as a pause maneuver or a plateau maneuver. They are separated in this discussion for clarity purposes.

In order to stabilize the pressure and flow to achieve no flow between the circuit and the patient, if there are leaks in the tubing system these leaks are compensated for by the ventilator. Thus, in order to stabilize the flow the ventilator provides a leak-compensation flow that is equal to the amount of leakage from the system estimated by the leakage model at the stable pressure.

In practice, the stabilization of the pressure and flow is an iterative process in which the ventilator monitors the pressure and adjusts delivered flow until the pressure and flow stabilize at the point where the pressure and flow correspond to a solution to the leak model and the lung flow is practically zero, i.e., the current flow provided by the ventilator is the leakage flow determined from the model using the current pressure. In an embodiment, for a flow and pressure to be considered stable, a certain acceptable error may be allowed between the calculated leakage (calculated based on the current pressure) and the actual measured flow. Such an error may be predetermined amount or range based on an absolute difference between delivery and calculated flow or pressure or relative difference (e.g., calculated flow within x% of actual stable flow at a given pressure). Various methods for stabilizing pressure and flow in a ventilation tubing system are known in the art and any suitable method may be adapted for use in conjunction with the technology described in this disclosure.

When attempting to stabilize the pressure and flow during the stabilization operation **506**, the leakage model may be used to increase the speed of the stabilization through a prediction of the likely resulting leakage flow at different pressures. This information may be used to determine a more accurate initial starting point for the stabilization and determine more accurate selection of adjustments to be made in order to more quickly converge on the stable pressure and flow.

After a stable pressure has been achieved, in the embodiment shown a maintain stable condition operation **508** is performed. The maintain stable condition operation **508** may maintain the stable pressure and stable, leak-compensating flow for a predetermined period of time such as 25-200 milliseconds or more preferably between 50-100 milliseconds. During the operation **458**, the drop in pressure over the period of the maneuver may be monitored to ensure that it is within some acceptable performance threshold. If it is not, the ventilator may resume attempting to stabilize the pressure and flow or may abort the method and attempt the method **500** at the end of the next inspiratory phase.

The stable pressure observed during the maintain stable condition operation **508** is then used to calculate the leak-compensated compliance in a lung compliance calculation operation **510**. The pressure value used may be an actual pressure or an average pressure observed over the maneuver period. Alternatively, different values derived from or based on the observed stable pressure may be calculated and used depending on the data required by the particular respiratory mechanics model being utilized.

In addition to using the stable pressure obtained during the pause maneuver, the compliance calculation operation **510** further utilizes leak-compensated lung flow and leak-compensated net lung volume when performing the calculation.

As discussed above, any suitable model for calculating lung compliance may be used. For example, in an embodiment of the compliance calculation operation **510** compliance is calculated using the following simple model:

Stable pressure = Leak-Compensated Net Lung Volume/Compliance.

or, stated a different way,

Compliance = Leak-Compensated Net Lung Volume/ Stable pressure.

By using a leak-compensated value for lung volume and a stable pressure determined while compensating for leaks in the patient circuit, a more accurate leak-compensated lung compliance is estimated.

The leak-compensated compliance then may be used in a subsequent operation to determine a leak-compensated lung resistance. In the embodiment of the method **500** shown, this is illustrated by optional resistance calculation operation **512**. In an embodiment of the resistance calculation operation **512** after the leak-compensated compliance has been determined, a resistance model that calculates lung resistance based on pressure, flow and compliance may be used to calculate resistance. An example of one such resistance model is as follows:

$$P(t_2)-P(t_1) = (V(t_2)-V(t_1))/C + R*(Q(t_2)-Q(t_1))$$

In which $t_1$ and $t_2$ are different times during a breath, $P(t)$ is the airway pressure at time t, $V(t)$ is the delivered lung volume at time t, C is the lung compliance, R is the lung resistance and $Q(t)$ is the net lung flow at time t. In the resistance model provided above, leak-compensated lung flow, leak-compensated net lung volume and leak-compensated lung compliance are utilized to obtain a leak-compensated resistance. This computation may be performed repeatedly over several appropriate time windows and combined together (e.g., by an averaging method) to generate an estimate for lung resistance. Also, lung resistance may be determined from the leak-compensated exhalation flow waveform subsequent to the inspiratory pause maneuver using algorithms for resistance estimation under no leak conditions.

In an alternative embodiment of method **500**, if it is determined that the ventilator has relatively low leakage, the lung compliance calculation operation **510** may forego the use of leak-compensated lung flow and net lung volume but still utilize the stable pressure determined through the provision of a leak-compensating flow during the pause maneuver. Lung compliance calculated in this fashion is still considered leak-compensated as the stable pressure was determined by compensating for leakage during the pause maneuver when generating the stable pressure.

**Leak-Compensation of Dynamic Determination of Lung Compliance and Resistance**

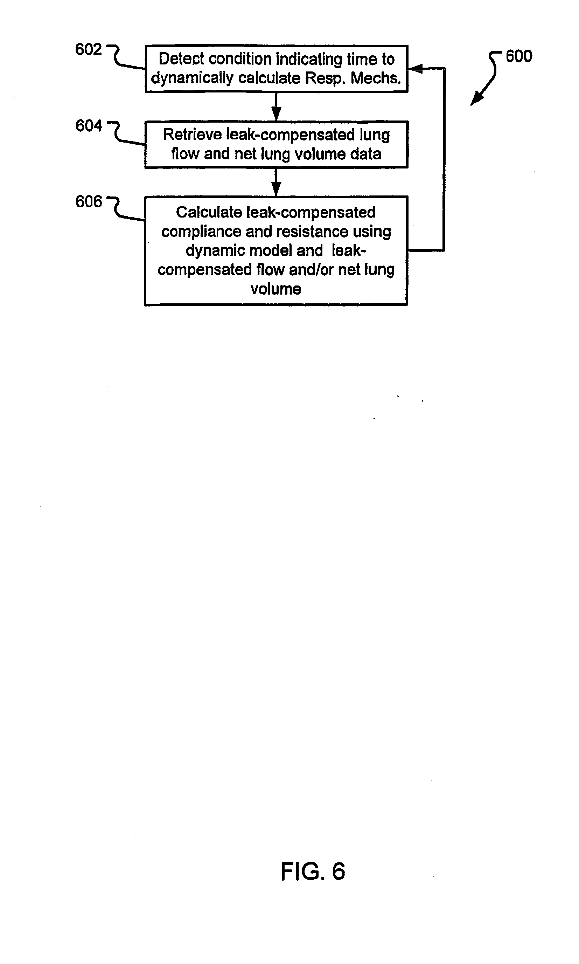

FIG. 6 illustrates an embodiment of a method for dynamically estimating respiratory mechanics of patient. This may be used as an alternative method of calculating lung compliance and resistance, thereby obviating the need to perform respiratory maneuvers during PA ventilation.

In the embodiment shown, the ventilator is providing respiratory gas to a patient in accordance with some mode of operation such as a mandatory mode or a pressure assist or support or PA mode, as is well known in the art. During operation, the ventilator detects a condition that indicates that it is time to estimate the respiratory mechanics of the patient. This is illustrated by the detection operation **602**. In an embodiment, the condition detected may be a command entered by the operator of the ventilator via selection of a button or other interface element on a user interface of the ventilator. Alternatively, the ventilator may perform the dynamic estimation automatically such as during every breath, after a predetermined period of time or after the detection of some occurrence such as once every 100 breaths or upon detection of certain flow conditions.

Following determination that it is time to calculate the dynamic respiratory mechanics, the method **600** retrieves and/or calculates leak-compensated lung flow and leak-compensated net lung volume as necessary depending on whether the leak-compensated data already exists or not. For example, in an embodiment the leak-compensated lung flows for each sampling period may be available but the leak-compensated net lung volume may only be available "as needed" by calculating it from the compensated lung flow data.

The method **600** then calculates the leak-compensated respiratory mechanics in a calculation operation **606**. The leak-compensated respiratory mechanics are calculated from a predetermined dynamic respiratory mechanics model using the leak-compensated lung flows, leak-compensated net lung volume(s) and pressure in order to obtain estimates of dynamic compliance and dynamic resistance that are compensated for the leaks in the tubing system. The method **600** is then repeated as necessary.

Any respiratory mechanics model may be used as long as the model may be adapted to be used in a dynamic calculation, that is without interrupting the ventilation of the patient. Many such models are known in the art, some requiring iterative solutions of a set of multiple equations using data obtained over of a period of time.

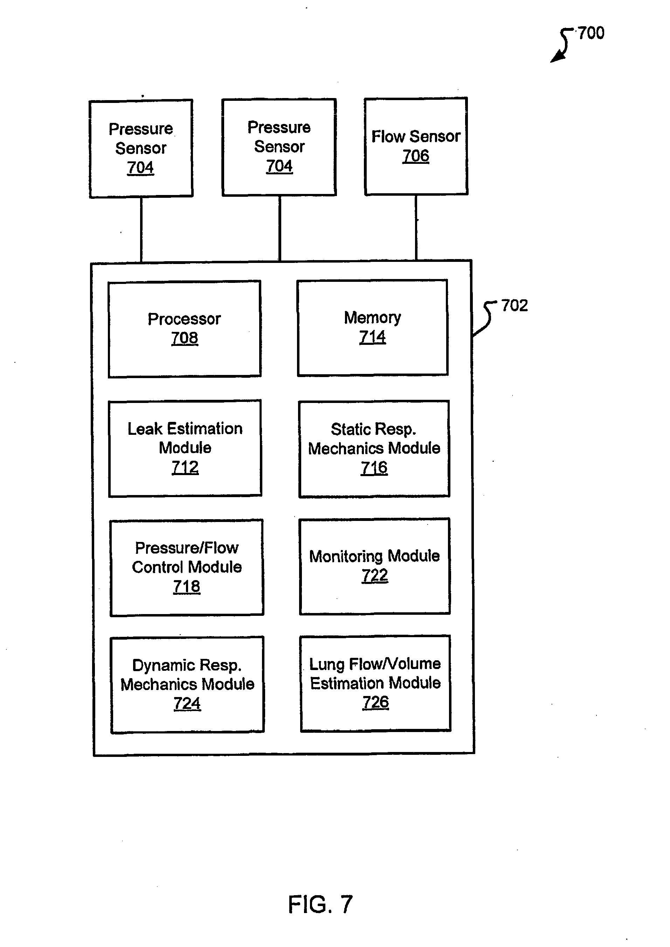

FIG. 7 illustrates a functional block diagram of modules and other components that may be used in an embodiment of ventilator that compensates for elastic and rigid orifice sources of leaks when determining patient respiratory mechanics. In the embodiment shown, the ventilator **700** includes pressure sensors **706** (two are shown placed at different locations in the system), flow sensors (one is shown), and a ventilator control system **702**. The ventilator control system **702** controls the operation of the ventilator and includes a plurality of modules described by their function. In the embodiment shown, the ventilator control system **702** includes a processor **708**, memory **714** which may include mass storage as described above, a leak estimation module **712** incorporating a parametric leak model accounting for both elastic and rigid orifice leak sources such as that described in U.S. Provisional Application 61/041,070 previously discussed herein, a leak-compensated static respiratory mechanics module **716**, a pressure and flow control module **718**, a monitoring module **722**, a leak model module **720**, a leak-compensated dynamic respiratory mechanics module **724**, and a leak-compensated lung flow and volume estimation module **726**. The processor **708** and memory **716** have been discussed above. Each of the other modules will be discussed in turn below.

The main functions of the ventilator such as receiving and interpreting operator inputs and providing therapy via changing pressure and flow of gas in the ventilator circuit are performed by the control module **718.** In the context of the methods and systems described herein, the module **718** will perform one or more actions upon the determination that a patient receiving therapy is inhaling or exhaling.

In the embodiment described herein, the control module **718** determines and provides the appropriate amount of ventilation when in PA ventilation mode. This may include calculating patient effort and, based on the patient effort and the support setting, determining the appropriate amount of ventilation, i.e., the pressure and/or flow to provide to the patient. This may include performing one or more calculations based on leak-compensated lung flow, leak-compensated lung volume, leak-compensated lung compliance and leak-compensated lung resistance. PA ventilation is based on an estimation of patient's respiratory effort, therefore, patient effort may be first calculated and then the amount of ventilation (desired pressure reference) calculated therefrom.

The static calculation of respiratory mechanics is performed by the leak-compensated static respiratory mechanics module **716**. The module **716** utilizes one or more respiratory models suitable for static determination of respiratory mechanics and one or more embodiments of the method **400** described above to calculate leak-compensated respiratory mechanics such as lung compliance and lung resistance. The module **716** uses leak-compensated values for one or both of lung flows and net lung volume. Leak-compensated values may be retrieved if they have already been calculated or may be calculated as needed from leakage information received from the leak-compensated lung flow and net lung volume estimation module **726**. When calculating static respiratory mechanics, the module **716** may control the operation of the ventilator so that a pause maneuver is performed when required. Alternatively, some or all of the actions required in a pause maneuver may be controlled by the control module **718** in response to a respiratory mechanics calculation request and the data obtained during the maneuver provided to the static respiratory mechanics module **716**.

The dynamic calculation of respiratory mechanics is performed by the leak-compensated dynamic respiratory mechanics module **724**. The module **724** utilizes one or more dynamic respiratory models and one or more embodiments of the method **500** described above to calculate leak-compensated respiratory mechanics such as lung compliance and lung resistance. The module **724** uses leak-compensated values for one or both of lung flows and net lung volume. Leak-compensated values may be retrieved if they have already been calculated or may be calculated from leakage information received from the leak-compensated lung flow and net lung volume estimation module **726**.

The current conditions in the ventilation system are monitored by the monitoring module **722**. This module **722** collects the data generated by the sensors **704, 706** and may also perform certain calculations on the data to make the data more readily usable by other modules or may process the current data and or previously acquired data or operator input to derive auxiliary parameters or attributes of interest. In an embodiment, the monitoring module **722** receives data and provides it to each of the other modules in the ventilator control system **702** that need the current pressure or flow data for the system.

In the embodiment shown, compensated lung flows are calculated by the lung flow module **726**. The lung flow module **726** uses a quantitative model for lung flow of the patient during both inhalation and exhalation and from this characterization and pressure and flow measurements generates an estimate for instantaneous lung flow. In an embodiment, lung flow may be simply determined based on subtracting the estimated leak flow and measured outflow via the expiratory limb from the flow into the inspiratory limb, thereby generating a leak-compensated net flow into (or out of) the lung. The lung flow module **726** may or may not also calculate an ongoing leak-compensated net lung volume during a patient's breath as described above. Compression in the circuits and accessories may also be accounted for to improve the accuracy of estimated lung flow.

The leak model parameters are generated by the leak estimation module **712** which creates one or more quantitative mathematical models, equations or correlations that uses pressure and flow observed in the ventilation system over regular periods of respiratory cycles (inhalation and exhalation) and apply physical and mathematical principles derived from mass balance and characteristic waveform settings of ventilation modalities (regulated pressure or flow trajectories) to derive the parameters of the leak model incorporating both rigid and elastic (variable pressure-dependent) orifices. In an embodiment, the mathematical model may be a model such as:

$$Q_{inelastic} = R_1 * P_i^x$$

$$Q_{elastic} = R_2 * P_i^y$$

wherein $Q_{elastic}$ is the instantaneous leak flow due to elastic leaks in the ventilation system, $Q_{inelastic}$ is the instantaneous leak flow due to inelastic leaks in the ventilation system, $R_1$ is the inelastic leak constant, $R_2$ is the elastic, leak constant, $P_i$ is the current or instantaneous pressure measurement, x is an exponent for use when determining the inelastic leak and y is an exponent different than x for use when determining the elastic leak. The group $R_1 * P_i^x$ represents flow through an orifice of fixed size as a function of instantaneous pressure $P_i$ and the group $R_2 * P_i^y$ represents flow through a different orifice that varies in size based on the instantaneous pressure. The equations above presuppose that there will always be an elastic component and an inelastic component of leakage from the ventilation system. In the absence of an elastic component or a leak source of varying size, $R_2$ would turn out be zero.

In the embodiment shown, the current or instantaneous elastic leak is calculated by the leak estimation module **712**. The calculation is made using the elastic leak portion of the leak model developed by the leak estimation module **712** and the pressure data obtained by the monitoring module **722**. The leak estimation module **712** may calculate a new instantaneous elastic leak flow or volume for each pressure sample taken (i.e., for each sampling period) by the monitoring module **722**. The calculated elastic leak may then be provided to any other module as needed.

In the embodiment shown, the current or instantaneous inelastic leak is also calculated by the leak estimation module **712**. The calculation is made using the inelastic leak portion of the leak model and the pressure data obtained by the monitoring module **722**. The leak estimation module **712** may calculate a new instantaneous inelastic leak flow or volume for each pressure sample taken (i.e., for each sampling period) by the monitoring module **722.** The calculated inelastic leak may then be provided to any other module as needed.

The system **700** illustrated will compensate lung flow for leaks due to elastic and inelastic leaks in the ventilation system. Furthermore, the system may perform a dynamic compensation of lung flow based on the changing leak conditions of the ventilation system and the instantaneous pressure and flow measurements. The system then compensates the respiratory mechanics calculations based on the estimated leakage in the system. By compensating for the inelastic as well as the elastic components of dynamic leaks, the medical ventilator can more accurately and precisely estimate the respiratory mechanics of a patient including estimating the lung compliance and lung resistance.

It will be clear that the systems and methods described herein are well adapted to attain the ends and advantages mentioned as well as those inherent therein. Those skilled in the art will recognize that the methods and systems within this specification may be implemented in many manners and as such is not to be limited by the foregoing exemplified embodiments and examples. For example, the operations and steps of the embodiments of methods described herein may be combined or the sequence of the operations may be changed while still achieving the goals of the technology. In addition, specific functions and/or actions may also be allocated in such as a way as to be performed by a different module or method step without deviating from the overall disclosure. In other words, functional elements being performed by a single or multiple components, in various combinations of hardware and software, and individual functions can be distributed among software applications. In this regard, any number of the features of the different embodiments described herein may be combined into one single embodiment and alternate embodiments having fewer than or more than all of the features herein described are possible.

While various embodiments have been described for purposes of this disclosure, various changes and modifications may be made which are well within the scope of the technology described herein. For example, the systems and methods described herein could be adapted to automatically determine leak-compensated resistance and/or compliance and initiate an alarm if the leak-compensated values are outside of a specified range for predetermined leakage values, thus eliminating false resistance and compliance alarms due to changes in leakage. Numerous other changes may be made which will readily suggest themselves to those skilled in the art.

**Claims**

1. A computer program module that when executed in a ventilation system during delivery of proportional assist ventilation causes the ventilation system to perform steps comprising:

   monitoring an instantaneous flow in the ventilation system based on one or more measurements of pressure and flow in the ventilation system (402);
   modeling leakage from the ventilation system as a first leakage component through a first orifice of a fixed size and a second leakage component through a second orifice of a varying size (404), wherein the first and second leakage components are different functions of instantaneous pressure in the ventilation system;
   estimating a leak-compensated instantaneous lung flow (406) of gas inhaled or exhaled based on the one or more measurements, the first leakage component and second leakage component;
   using the leak compensated lung flow and a predetermined respiratory mechanics model to estimate a leak-compensated lung compliance and a leak-compensated lung resistance (414); and
   calculating a pressure to be delivered based on the leak- compensated lung flow, the leak-compensated lung compliance and the leak- compensated lung resistance.

2. The computer program module of claim 1 wherein the ventilation system uses a dynamic respiratory mechanics model and the computer program module is executable to cause the ventilation system to perform further steps comprising:

   detecting a condition indicating that leak-compensated lung compliance should be calculated;
   retrieving one or more previously estimated leak-compensated instantaneous lung flows; and
   estimating a leak-compensated lung compliance and a leak-compensated lung resistance using the dynamic respiratory mechanics model and the retrieved one or more previously estimated leak-compensated instantaneous lung flows.

3. The computer program module of claim 2 wherein the dynamic respiratory mechanics model requires input of a lung volume change over a predetermined time period and wherein the computer program module is executable to cause the ventilation system to perform the step of:

   calculating a leak-compensated lung volume change for the predetermined time period based on leak-compensated lung flows associated with the predetermined time period.

4. The computer program module of claim 1 wherein the computer program module is executable to calculate at least the leak- compensated lung compliance and to cause the ventilation system to perform further steps comprising:

   calculating a leak-compensated lung volume based on the leak- compensated lung flow during an inspiratory phase;
   stabilizing the delivery of gas so that the medical ventilator delivers a stable flow of gas at a stable pressure, wherein the stable flow and stable pressure are determined based on the first leakage component and second leakage component at the stable pressure;
   maintaining the stable flow of gas at the stable pressure for at least a predetermined time interval; and
   calculating the leak-compensated lung compliance based on the leak-compensated lung volume and the stable pressure.

5. The computer program module of claim 4 wherein the stabilizing further comprises:

   stabilizing the delivery of gas by adjusting the instantaneous flow of gas to be within a predetermined amount of the first leakage component and second leakage component at the instantaneous pressure

   and/or wherein the stabilizing further comprises:

   stabilizing the delivery of gas so that the lung flow is practically zero and/or wherein the stabilizing and maintaining operations are performed at the end of the inspiratory phase.

6. The computer program module of claim 4: wherein the computer program module is executable to cause the ventilation system to perform the step of:

calculating a leak-compensated lung resistance based on the leak-compensated lung compliance and one or more of a previously calculated leak-compensated lung flow, a previously calculated leak-compensated lung volume and a previously measured pressure.

7. The computer program module of claim 1 wherein the computer program module is executable to calculate at least the lung compliance and to cause the ventilation system to perform further steps comprising:

calculating a leak-compensated lung volume based on the leak-compensated lung flow during an inspiratory phase and expiratory phase;
stabilizing the delivery of gas so that the medical ventilator delivers a stable flow of gas at a stable pressure, wherein the stable flow and stable pressure are determined based on the first leakage component and second leakage component at the stable pressure;
maintaining the stable flow of gas at the stable pressure for at least a predetermined time interval; and
calculating the leak-compensated lung compliance based on the leak-compensated lung volume and the stable pressure
and/or first calculating a leak-compensated lung compliance and then, based on the leak-compensated lung compliance, calculating a leak-compensated lung resistance.

8. A computer program module that when executed in a ventilation system during delivery of gas causes a medical ventilator to perform steps for ventilation tubing system compensation comprising:

receiving a support setting identifying an amount of proportional assistance to provide;
identifying an inelastic leakage in the ventilation tubing system as a first function of at least one of a pressure measurement and a flow measurement in the ventilation tubing system, wherein the inelastic leakage is modelled as a first leakage component through a first orifice of a fixed size;
identifying an elastic leakage in the ventilation tubing system as a second function of at least one of the pressure measurement and the flow measurement in the ventilation tubing system, wherein the elastic leakage is modelled as a second leakage component through a second orifice of a varying size;
estimating circuit compliance and circuit resistance of the ventilation tubing system;
estimating a lung compliance and a lung resistance based on the inelastic leakage, the elastic leakage, the circuit compliance, circuit resistance and the at least one of the pressure measurement and the flow measurement in the ventilation tubing system;
delivering ventilation based on patient effort and the support setting, wherein the patient effort is determined from the inelastic leakage, the elastic leakage, the lung compliance, the lung resistance and the at least one of the pressure measurement and the flow measurement in the ventilation tubing system.

9. The computer program module of claim 8 wherein estimating lung compliance comprises:

generating a plurality of leak-compensated lung flows associated with a period of time based on the elastic leakage and at least one of pressure measurements and the flow measurements associated with the period of time;
generating a leak-compensated net lung volume for the period of time based on the plurality of leak-compensated lung flows; and
calculating lung compliance using the leak-compensated net lung volume,

or wherein estimating lung compliance and lung resistance further comprises:

generating a plurality of leak-compensated lung flows associated with a period of time based on the elastic leakage and at least one of pressure measurements and the flow measurements associated with the period of time;
generating a leak-compensated net lung volume for the period of time based on the plurality of leak-compensated lung flows; and
calculating lung compliance and lung resistance using the leak-compensated net lung volume, the plurality of leak-compensated lung flows for the period of time and the pressure measurements associated with the period of time.

10. A pressure support system comprising:

a pressure generating system adapted to generate a flow of breathing gas;

a ventilation tubing system including a patient interface device for connecting the pressure generating system to a patient;

one or more sensors (704) operatively coupled to the pressure generating system or the ventilation tubing system, each sensor capable of generating an output indicative of a pressure of the breathing gas;

a leak estimation module (712) that identifies leakage in the ventilation tubing system, wherein the leak estimation module is configured to model leakage from the ventilation system as an inelastic leakage component through a first orifice of a fixed size and an elastic leakage component through a second orifice of a varying size;

a respiratory mechanics calculation module (724) that generates a leak-compensated lung compliance and a leak-compensated lung resistance based on the leakage and at least one output indicative of a pressure of the breathing gas; and

a proportional assistance ventilation module that causes the pressure generating system to provide ventilation to the patient based on patient effort and a support setting, wherein the patient effort is determined from the leakage, the leak-compensated lung compliance and the leak-compensated lung resistance.

11. The system of claim 10 wherein the respiratory mechanics module is a static respiratory mechanics module that uses a static respiratory mechanics model to determine a leak-compensated static lung compliance and a leak-compensated static lung resistance based on a leak-compensated lung flow and an output of at least one sensor, or wherein the respiratory mechanics module is a dynamic respiratory mechanics module that uses a dynamic respiratory mechanics model to determine at least one of a leak-compensated dynamic lung compliance and a leak-compensated dynamic lung resistance based on the leak-compensated lung flow and an output of at least one sensor.

12. The system of claim 10 wherein the compensation module further generates the leak-compensated lung flow based on the inelastic leakage.

13. The system of claim 12 further comprising:

a pressure monitoring module that monitors at least one output of the one or more sensors and provides data indicative of the pressure of the breathing gas to the leak estimation module.

**Patentansprüche**

1. Computerprogrammmodul, das bei Ausführung in einem Beatmungssystem während der Zufuhr einer proportional unterstützten Beatmung das Beatmungssystem veranlasst, Schritte durchzuführen, die umfassen:

Überwachen eines momentanen Durchflusses im Beatmungssystem basierend auf einer oder mehreren Druck- und Durchflussmessungen im Beatmungssystem (402);

Modellieren einer Leckage aus dem Beatmungssystem als eine erste Leckagekomponente durch eine erste Öffnung fester Größe und eine zweite Leckagekomponente durch eine zweite Öffnung variierender Größe (404), wobei die erste und zweite Leckagekomponente unterschiedliche Funktionen des momentanen Drucks im Beatmungssystem sind;

Schätzen eines leckkompensierten momentanen Lungendurchflusses (406) inhalierten oder exhalierten Gases basierend auf der einen oder den mehreren Messungen, der ersten Leckagekomponente und der zweiten Leckagekomponente;

Verwenden des leckkompensierten Lungendurchflusses und eines vorgegebenen Atmungsmechanikmodells zum Schätzen einer leckkompensierten Lungenkomplianz und eines leckkompensierten Lungenwiderstands (414); und

Berechnen eines Drucks, der basierend auf dem leckkompensierten Lungendurchfluss, der leckkompensierten Lungenkomplianz und dem leckkompensierten Lungenwiderstand zuzuführen ist.

2. Computerprogrammmodul nach Anspruch 1, wobei das Beatmungssystem ein dynamisches Atmungsmechanikmodell verwendet und das Computerprogrammmodul ausführbar ist, um das Beatmungssystem zu veranlassen, weitere Schritte durchzuführen, die umfassen:

Erfassen eines Zustands, welcher angibt, dass die leckkompensierte Lungenkomplianz berechnet werden sollte;
Abrufen von einem oder mehreren zuvor geschätzten leckkompensierten momentanen Lungendurchflüssen; und
Schätzen einer leckkompensierten Lungenkomplianz und eines leckkompensierten Lungenwiderstands unter Verwendung des dynamischen Atmungsmechanikmodells und des/der abgerufenen einen oder mehreren zuvor geschätzten leckkompensierten momentanen Lungendurchflüsse.

3. Computerprogrammmodul nach Anspruch 2, wobei das dynamische Atmungsmechanikmodell die Eingabe einer Lungenvolumenänderung über eine vorgegebene Zeitspanne hinweg erfordert und wobei das Computerprogrammmodul ausführbar ist, um das Beatmungssystem zu veranlassen, den Schritt durchzuführen:

Berechnen einer leckkompensierten Lungenvolumenänderung für die vorgegebene Zeitspanne basierend auf den leckkompensierten Lungendurchflüssen, die der vorgegebenen Zeitspanne zugeordnet sind.

4. Computerprogrammmodul nach Anspruch 1, wobei das Computerprogrammmodul ausführbar ist, um zumindest die leckkompensierte Lungenkomplianz zu berechnen und das Beatmungssystem zu veranlassen, weitere Schritte durchzuführen, die umfassen:

Berechnen eines leckkompensierten Lungenvolumens basierend auf dem leckkompensierten Lungendurchfluss während einer Einatmungsphase;
Stabilisieren der Gaszufuhr, so dass das medizinische Beatmungsgerät einen stabilen Gasdurchfluss mit stabilem Druck zuführt, wobei der stabile Durchfluss und der stabile Druck basierend auf der ersten Leckagekomponente und der zweiten Leckagekomponente bei dem stabilen Druck bestimmt werden;
Aufrechterhalten des stabilen Gasdurchflusses bei dem stabilen Druck für zumindest ein vorgegebenes Zeitintervall; und
Berechnen der leckkompensierten Lungenkomplianz basierend auf dem leckkompensierten Lungenvolumen und dem stabilen Druck.

5. Computerprogrammmodul nach Anspruch 4, wobei das Stabilisieren ferner umfasst:

Stabilisieren der Gaszufuhr durch Einstellen des momentanen Gasdurchflusses, so dass er in einer vorgegebenen Menge der ersten Leckagekomponente und der zweiten Leckagekomponente bei dem momentanen Druck liegt,

und/oder wobei das Stabilisieren ferner umfasst:

Stabilisieren der Gaszufuhr, so dass der Lungendurchfluss praktisch null ist und/oder wobei die Stabilisierungs- und Aufrechterhaltungsvorgänge am Ende der Einatmungsphase durchgeführt werden.

6. Computerprogrammmodul nach Anspruch 4, wobei das Computerprogrammmodul ausführbar ist, um das Beatmungssystem zu veranlassen, den Schritt durchzuführen:

Berechnen eines leckkompensierten Lungenwiderstands basierend auf der leckkompensierten Lungenkomplianz und einem oder mehreren eines zuvor berechneten leckkompensierten Lungendurchflusses, eines zuvor berechneten leckkompensierten Lungenvolumens und eines zuvor gemessenen Drucks.

7. Computerprogrammmodul nach Anspruch 1, wobei das Computerprogrammmodul ausführbar ist, um zumindest die Lungenkomplianz zu berechnen und das Beatmungssystem zu veranlassen, weitere Schritte durchzuführen, die umfassen:

Berechnen eines leckkompensierten Lungenvolumens basierend auf dem leckkompensierten Lungendurchfluss während einer Einatmungsphase und einer Ausatmungsphase;
Stabilisieren der Gaszufuhr, so dass das medizinische Beatmungsgerät einen stabilen Gasdurchfluss mit stabilem Druck zuführt, wobei der stabile Durchfluss und der stabile Druck basierend auf der ersten Leckagekomponente und der zweiten Leckagekomponente bei dem stabilen Druck bestimmt werden;
Aufrechterhalten des stabilen Gasdurchflusses bei dem stabilen Druck für zumindest ein vorgegebenes Zeitintervall; und

Berechnen der leckkompensierten Lungenkomplianz basierend auf dem leckkompensierten Lungenvolumen und dem stabilen Druck

und/oder zunächst Berechnen einer leckkompensierten Lungenkomplianz und dann Berechnen eines leckkompensierten Lungenwiderstands basierend auf der leckkompensierten Lungenkomplianz.

8. Computerprogrammmodul, welches bei Ausführung in einem Beatmungssystem während der Zufuhr von Gas ein medizinisches Beatmungsgerät veranlasst, Schritte zur Beatmungsschlauchsystemkompensation durchzuführen, die umfassen:

Empfangen einer Hilfseinstellung, die eine Menge der proportionalen Unterstützung identifiziert, die bereitzustellen ist;

Identifizieren einer unelastischen Leckage in dem Beatmungsschlauchsystem als erste Funktion einer Druckmessung und/oder einer Durchflussmessung in dem Beatmungsschlauchsystem, wobei die unelastische Leckage als erste Leckagekomponente durch eine erste Öffnung fester Größe modelliert ist;

Identifizieren einer elastischen Leckage in dem Beatmungsschlauchsystem als zweite Funktion der Druckmessung und/oder der Durchflussmessung in dem Beatmungsschlauchsystem, wobei die elastische Leckage als zweite Leckagekomponente durch eine zweite Öffnung variierender Größe modelliert ist;

Schätzen der Kreislaufnachgiebigkeit und des Kreislaufwiderstands des Beatmungsschlauchsystems;

Schätzen einer Lungenkomplianz und eines Lungenwiderstands basierend auf der unelastischen Leckage, der elastischen Leckage, der Kreislaufkomplianz, dem Kreislaufwiderstand und der Druckmessung und/oder Durchflussmessung in dem Beatmungsschlauchsystem;

Zuführen einer Beatmung basierend auf dem vom Patienten geleisteten Aufwand und der Hilfseinstellung, wobei der vom Patienten geleistete Aufwand aus der unelastischen Leckage, der elastischen Leckage, der Lungenkomplianz, dem Lungenwiderstand und der Druckmessung und/oder Durchflussmessung im Beatmungsschlauchsystem bestimmt wird.

9. Computerprogrammmodul nach Anspruch 8, wobei das Schätzen der Lungenkomplianz umfasst:

Erzeugen mehrerer, einer Zeitspanne zugeordneter leckkompensierter Lungendurchflüsse basierend auf der elastischen Leckage und den Druckmessungen und/oder Durchflussmessungen, die der Zeitspanne zugeordnet sind;

Erzeugen eines leckkompensierten Nettolungenvolumens für die Zeitspanne basierend auf den mehreren leckkompensierten Lungendurchflüssen; und

Berechnen der Lungenkomplianz unter Verwendung des leckkompensierten Nettolungenvolumens,

oder wobei das Schätzen der Lungenkomplianz und des Lungenwiderstands ferner umfasst:

Erzeugen mehrerer, einer Zeitspanne zugeordneter leckkompensierter Lungendurchflüsse basierend auf der elastischen Leckage und den Druckmessungen und/oder Durchflussmessungen, die der Zeitspanne zugeordnet sind;

Erzeugen eines leckkompensierten Nettolungenvolumens für die Zeitspanne basierend auf den mehreren leckkompensierten Lungendurchflüssen; und

Berechnen einer Lungenkomplianz und eines Lungenwiderstands unter Verwendung des leckkompensierten Nettolungenvolumens, der mehreren leckkompensierten Lungendurchflüsse für die Zeitspanne und der Druckmessungen, die der Zeitspanne zugeordnet sind.

10. Druckhilfssystem mit:

einem Druckerzeugungssystem, das dazu ausgelegt ist, einen Atmungsgasdurchfluss zu erzeugen;

einem Beatmungsschlauchsystem einschließlich einer Patientenschnittstellenvorrichtung zum Verbinden des Druckerzeugungssystems mit einem Patienten;

einem oder mehreren Sensoren (704), die betriebsfähig mit dem Druckerzeugungssystem oder dem Beatmungsschlauchsystem verbunden sind, wobei jeder Sensor eine Ausgabe zu erzeugen vermag, die den Druck des Atmungsgases angibt;

einem Leckschätzungsmodul (712), das eine Leckage im Beatmungsschlauchsystem identifiziert, wobei das Leckschätzungsmodul dazu ausgebildet ist, eine Leckage aus dem Beatmungssystem als unelastische Leckagekömponente durch eine erste Öffnung fester Größe und eine elastische Leckagekomponente durch eine zweite Öffnung variierender Größe zu modellieren;

einem Atmungsmechanik-Berechnungsmodul (724), das eine leckkompensierte Lungenkomplianz und einen leckkompensierten Lungenwiderstand basierend auf der Leckage und zumindest einer Ausgabe erzeugt, die einen Druck des Atmungsgases angibt; und

einem Proportionalunterstützungs-Beatmungsmodul, welches das Druckerzeugungssystem veranlasst, dem Patienten eine Beatmung basierend auf dem vom Patienten geleisteten Aufwand und einer Hilfseinstellung bereitzustellen, wobei der vom Patienten geleistete Aufwand aus der Leckage, der leckkompensierten Lungenkomplianz und dem leckkompensierten Lungenwiderstand bestimmt wird.

11. System nach Anspruch 10, wobei das Atmungsmechanikmodul ein statisches Atmungsmechanikmodul ist, das ein statisches Atmungsmechanikmodul verwendet, um eine leckkompensierte statische Lungenkomplianz und einen leckkompensierten statischen Lungenwiderstand basierend auf einem leckkompensierten Lungendurchfluss und einer Ausgabe zumindest eines Sensors zu bestimmen,

oder wobei das Atmungsmechanikmodul ein dynamisches Atmungsmechanikmodul ist, das ein dynamisches Atmungsmechanikmodul verwendet, um eine leckkompensierte dynamische Lungenkomplianz und/oder einen leckkompensierten dynamischen Lungenwiderstand basierend auf dem leckkompensierten Lungendurchfluss und einer Ausgabe zumindest eines Sensors zu bestimmen.

12. System nach Anspruch 10, wobei das Kompensationsmodul ferner den leckkompensierten Lungendurchfluss basierend auf der unelastischen Leckage erzeugt.

13. System nach Anspruch 12, ferner mit:

einem Drucküberwachungsmodul, das zumindest eine Ausgabe des einen oder der mehreren Sensoren überwacht und dem Leckschätzungsmodul Daten bereitstellt, die den Druck des Atmungsgases angeben.

## Revendications

1. Module de programme informatique qui, lorsqu'il est exécuté dans un système de ventilation lors d'une délivrance de ventilation assistée proportionnelle, fait que le système de ventilation met en oeuvre des étapes comprenant :

la surveillance d'un débit instantané dans le système de ventilation sur la base d'une ou plusieurs mesure(s) de pression et de débit dans le système de ventilation (402) ;

la modélisation d'une fuite hors du système de ventilation comme une première composante de fuite à travers un premier orifice d'une taille fixe et une deuxième composante de fuite à travers un deuxième orifice d'une taille variable (404), dans lequel les première et deuxième composantes de fuite sont des fonctions différentes d'une pression instantanée dans le système de ventilation ;

l'estimation d'un débit pulmonaire instantané à compensation de fuite (406) de gaz inhalé ou exhalé sur la base de l'une ou des plusieurs mesure(s), de la première composante de fuite et de la deuxième composante de fuite ;

l'utilisation du débit pulmonaire à compensation de fuite et d'un modèle de mécanique respiratoire prédéterminé pour estimer une compliance pulmonaire à compensation de fuite et une résistance pulmonaire à compensation de fuite (414) ; et

le calcul d'une pression à délivrer sur la base du débit pulmonaire à compensation de fuite, de la compliance pulmonaire à compensation de fuite et de la résistance pulmonaire à compensation de fuite.

2. Module de programme informatique selon la revendication 1, dans lequel le système de ventilation utilise un modèle de mécanique respiratoire dynamique et le module de programme informatique est exécutable pour faire que le système de ventilation mette en oeuvre des étapes supplémentaires comprenant :

la détection d'une condition indiquant qu'une compliance pulmonaire à compensation de fuite doit être calculée ;

la récupération d'un ou plusieurs débit(s) pulmonaire(s) instantané(s) à compensation de fuite estimé(s) antérieurement ; et

l'estimation d'une compliance pulmonaire à compensation de fuite et d'une résistance pulmonaire à compensation de fuite en utilisant le modèle de mécanique respiratoire dynamique et l'un ou les plusieurs débit(s) pulmonaire(s) instantané(s) à compensation de fuite estimé(s) antérieurement récupérés.

3. Module de programme informatique selon la revendication 2, dans lequel le modèle de mécanique respiratoire dynamique requiert une entrée d'un changement de volume pulmonaire sur une période de temps prédéterminée et dans lequel le module de programme informatique est exécutable pour faire que le système de ventilation mette en oeuvre l'étape de :

calcul d'un changement de volume pulmonaire à compensation de fuite pour la période de temps prédéterminée sur la base de débits pulmonaires à compensation de fuite associés avec la période de temps prédéterminée.

4. Module de programme informatique selon la revendication 1, dans lequel le module de programme informatique est exécutable pour calculer au moins la compliance pulmonaire à compensation de fuite et pour faire que le système de ventilation mette en oeuvre des étapes supplémentaires comprenant :

le calcul d'un volume pulmonaire à compensation de fuite sur la base du débit pulmonaire à compensation de fuite pendant une phase d'inspiration ;
la stabilisation de la délivrance de gaz de façon à ce que le ventilateur médical délivre un débit de gaz stable à une pression stable, dans lequel le débit stable et la pression stable sont déterminés sur la base de la première composante de fuite et de la deuxième composante de fuite à la pression stable ;
le maintien du débit de gaz stable à la pression stable sur au moins un intervalle de temps prédéterminé ; et
le calcul de la compliance pulmonaire à compensation de fuite sur la base du volume pulmonaire à compensation de fuite et de la pression stable.

5. Module de programme informatique selon la revendication 4, dans lequel la stabilisation comprend en outre :

la stabilisation de la délivrance de gaz en ajustant le débit de gaz instantané de façon à ce qu'il soit à l'intérieur d'une quantité prédéterminée de la première composante de fuite et de la deuxième composante de fuite à la pression instantanée
et/ou dans lequel la stabilisation comprend en outre :

la stabilisation de la délivrance de gaz de façon à ce que le débit pulmonaire soit pratiquement zéro et/ou dans lequel des opérations de stabilisation et de maintien sont mises en oeuvre à la fin de la phase d'inspiration.

6. Module de programme informatique selon la revendication 4, dans lequel le module de programme informatique est exécutable pour faire que le système de ventilation mette en oeuvre l'étape de :

calcul d'une résistance pulmonaire à compensation de fuite sur la base de la compliance pulmonaire à compensation de fuite et d'un ou plusieurs parmi un débit pulmonaire à compensation de fuite calculé antérieurement, un volume pulmonaire à compensation de fuite calculé antérieurement et une pression mesurée antérieurement.

7. Module de programme informatique selon la revendication 1, dans lequel le module de programme informatique est exécutable pour calculer au moins la compliance pulmonaire et pour faire que le système de ventilation mette en oeuvre des étapes supplémentaires comprenant :

le calcul d'un volume pulmonaire à compensation de fuite sur la base du débit pulmonaire à compensation de fuite pendant une phase d'inspiration et une phase d'expiration ;
la stabilisation de la délivrance de gaz de façon à ce que le ventilateur médical délivre un débit de gaz stable à une pression stable, dans lequel le débit stable et la pression stable sont déterminés sur la base de la première composante de fuite et de la deuxième composante de fuite à la pression stable ;
le maintien du débit de gaz stable à la pression stable sur au moins un intervalle de temps prédéterminé ; et
le calcul de la compliance pulmonaire à compensation de fuite sur la base du volume pulmonaire à compensation de fuite et de la pression stable
et/ou d'abord le calcul d'une compliance pulmonaire à compensation de fuite et ensuite, sur la base de la compliance pulmonaire à compensation de fuite, le calcul d'une résistance pulmonaire à compensation de fuite.

8. Module de programme informatique qui, lorsqu'il est exécuté dans un système de ventilation lors d'une délivrance de gaz fait qu'un ventilateur médical met en oeuvre des étapes pour une compensation de système de tubage de ventilation comprenant :

la réception d'un réglage de soutien identifiant une quantité d'assistance proportionnelle à prévoir ;
l'identification d'une fuite non élastique dans le système de tubage de ventilation comme une première fonction d'au moins une parmi une mesure de pression et une mesure de débit dans le système de tubage de ventilation, dans lequel la fuite non élastique est modélisée comme une première composante de fuite à travers un premier orifice d'une taille fixe ;

l'identification d'une fuite élastique dans le système de tubage de ventilation comme une deuxième fonction d'au moins une parmi la mesure de pression et la mesure de débit dans le système de tubage de ventilation, dans lequel la fuite élastique est modélisée comme une deuxième composante de fuite à travers un deuxième orifice d'une taille variable ;

l'estimation d'une compliance de circuit et d'une résistance de circuit du système de tubage de ventilation ;

l'estimation d'une compliance pulmonaire et d'une résistance pulmonaire sur la base de la fuite non élastique, de la fuite élastique, de la compliance de circuit, de la résistance de circuit et de l'au moins une parmi la mesure de pression et la mesure de débit dans le système de tubage de ventilation ;

la délivrance d'une ventilation sur la base d'un effort du patient et du réglage de soutien, dans lequel l'effort du patient est déterminé à partir de la fuite non élastique, de la fuite élastique, de la compliance pulmonaire, de la résistance pulmonaire et de l'au moins une parmi la mesure de pression et la mesure de débit dans le système de tubage de ventilation.

9. Module de programme informatique selon la revendication 8, dans lequel l'estimation de la compliance pulmonaire comprend :

la génération d'une pluralité de débits pulmonaires à compensation de fuite associés avec une période de temps sur la base de la fuite élastique et d'au moins une parmi des mesures de pression et les mesures de débit associées avec la période de temps ;

la génération d'un volume pulmonaire net à compensation de fuite pour la période de temps sur la base de la pluralité de débits pulmonaires à compensation de fuite ; et

le calcul d'une compliance pulmonaire en utilisant le volume pulmonaire net à compensation de fuite,

ou dans lequel l'estimation d'une compliance pulmonaire et d'une résistance pulmonaire comprend en outre :

la génération d'une pluralité de débits pulmonaires à compensation de fuite associés avec une période de temps sur la base de la fuite élastique et d'au moins une parmi des mesures de pression et les mesures de débit associées avec la période de temps ;

la génération d'un volume pulmonaire net à compensation de fuite pour la période de temps sur la base de la pluralité de débits pulmonaires à compensation de fuite ; et

le calcul d'une compliance pulmonaire et d'une résistance pulmonaire en utilisant le volume pulmonaire net à compensation de fuite, la pluralité de débits pulmonaires à compensation de fuite pour la période de temps et les mesures de pression associées avec la période de temps.

10. Système de soutien de pression comprenant :

un système de génération de pression adapté à générer un débit de gaz respiratoire ;

un système de tubage de ventilation incluant un dispositif d'interface patient pour connecter le système de génération de pression à un patient ;

un ou plusieurs capteur(s) (704) opérationnellement couplé(s) au système de génération de pression ou au système de tubage de ventilation, chaque capteur apte à générer une sortie indicatrice d'une pression du gaz respiratoire ;

un module d'estimation de fuite (712) qui identifie une fuite dans le système de tubage de ventilation, dans lequel le module d'estimation de fuite est configuré pour modéliser une fuite hors du système de ventilation comme une composante de fuite non élastique à travers un premier orifice d'une taille fixe et une composante de fuite élastique à travers un deuxième orifice d'une taille variable ;

un module de calcul de mécanique respiratoire (724) qui génère une compliance pulmonaire à compensation de fuite et une résistance pulmonaire à compensation de fuite sur la base de la fuite et d'au moins une sortie indicatrice d'une pression du gaz respiratoire ; et

un module de ventilation à assistance proportionnelle qui fait que le système de génération de pression délivre une ventilation au patient sur la base d'un effort du patient et d'un réglage de soutien, dans lequel l'effort du patient est déterminé à partir de la fuite, de la compliance pulmonaire à compensation de fuite et de la résistance pulmonaire à compensation de fuite.

11. Système selon la revendication 10, dans lequel le module de mécanique respiratoire est un module de mécanique respiratoire statique qui utilise un modèle de mécanique respiratoire statique pour déterminer une compliance pulmonaire statique à compensation de fuite et une résistance pulmonaire statique à compensation de fuite sur la base d'un débit pulmonaire à compensation de fuite et d'une sortie d'au moins un capteur,
ou dans lequel le module de mécanique respiratoire est un module de mécanique respiratoire dynamique qui utilise un modèle de mécanique respiratoire dynamique pour déterminer au moins une parmi une compliance pulmonaire dynamique à compensation de fuite et une résistance pulmonaire dynamique à compensation de fuite sur la base du débit pulmonaire à compensation de fuite et d'une sortie d'au moins un capteur.

12. Système selon la revendication 10, dans lequel le module de compensation génère en outre le débit pulmonaire à compensation de fuite sur la base de la fuite non élastique.

13. Système selon la revendication 12, comprenant en outre :

un module de surveillance de pression qui surveille au moins une sortie du ou des capteur(s) et délivre des données indicatrices de la pression du gaz respiratoire au module d'estimation de fuite.

Fig. 1

Fig. 2

302 — Estimate ventilator tubing
compliance and resistance

⌐ 300

304 — Begin ventilatory support of patient/
Perform initialization

306 — Provide PA ventilation

307 — Calculate leakage and
leak-compensated lung flow
and volume

308 — Perform leak-compensated resp.
mech. maneuver

## FIG. 3

400

402 — Monitor pressure and flow

404 — Calculate instantaneous leakage from elastic and inelastic leaks

406 — Calculate leak-compensated instantaneous lung flow and revise net lung volume

408 — Calculate patient effort based on support settings, resp. mech. and leak-compensated lung flow

410 — Provide assistance based on support setting and patient effort

412 — Time to Calculate Resp. Mech.?    N

Wait until next sample period

Y

414 — Calculate lung compliance and/or lung resistance using net lung volume and/or lung flow

FIG. 4

502 — Receive direction to estimate compliance

⌐500

504 — Detect end of inhalation phase

506 — Stabilize pressure and flow based on estimated leakage

508 — Maintain stable pressure and flow and obtain pressure

510 — Calculate leak-compensated compliance based on leak-compensated net lung volume and stable pressure

512 — Calculate leak-compensated resistance based on leak-compensated compliance, leak-compensated volume and leak-compensated net flow

FIG. 5

602 — Detect condition indicating time to dynamically calculate Resp. Mechs.

600

604 — Retrieve leak-compensated lung flow and net lung volume data

606 — Calculate leak-compensated compliance and resistance using dynamic model and leak-compensated flow and/or net lung volume

FIG. 6

⌐700

┌──────────────┐    ┌──────────────┐    ┌──────────────┐
│  Pressure    │    │  Pressure    │    │ Flow Sensor  │
│  Sensor      │    │  Sensor      │    │   706        │
│  704         │    │  704         │    │              │
└──────────────┘    └──────────────┘    └──────────────┘

⌐702

┌────────────────────────────────────────────────────┐
│                                                      │
│  ┌──────────────┐         ┌──────────────┐          │
│  │  Processor   │         │   Memory     │          │
│  │  708         │         │   714        │          │
│  └──────────────┘         └──────────────┘          │
│                                                      │
│  ┌──────────────┐         ┌──────────────┐          │
│  │  Leak        │         │  Static Resp.│          │
│  │  Estimation  │         │  Mechanics   │          │
│  │  Module      │         │  Module      │          │
│  │  712         │         │  716         │          │
│  └──────────────┘         └──────────────┘          │
│                                                      │
│  ┌──────────────┐         ┌──────────────┐          │
│  │ Pressure/Flow│         │  Monitoring  │          │
│  │ Control      │         │  Module      │          │
│  │ Module       │         │  722         │          │
│  │ 718          │         │              │          │
│  └──────────────┘         └──────────────┘          │
│                                                      │
│  ┌──────────────┐         ┌──────────────┐          │
│  │ Dynamic Resp.│         │ Lung Flow/   │          │
│  │ Mechanics    │         │ Volume       │          │
│  │ Module       │         │ Estimation   │          │
│  │ 724          │         │ Module 726   │          │
│  └──────────────┘         └──────────────┘          │
│                                                      │
└────────────────────────────────────────────────────┘

FIG. 7

# EP 2 259 820 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 0010634 A
- WO 2004084980 A
- DE 19808543
- US 61041070 A

28